# EUROPÄISCHE PATENTANMELDUNG

(11) **EP 1 136 868 A1**
(43) Veröffentlichungstag der Anmeldung: **26.09.2001**
(21) Anmeldenummer: 01106030.8
(22) Anmeldetag: 12.03.2001
(51) Int. Cl.: G02C 13/00

(54) **Verfahren zur indirekten Anpassung einer ausgewählten Brille und Schablone zur Durchführung**

(30) Priorität: 14.03.2000 DE 20004680 U
(71) Anmelder: Dehmer, Dietrich, 63619 Bad Orb (DE)
(72) Erfinder: Dehmer, Dietrich, 63619 Bad Orb (DE)
(74) Vertreter: Wolf, Günter, Dipl.-Ing.

(57) **Zusammenfassung**

Die Erfindung betrifft ein Verfahren zur indirekten Anpassung einer ausgewählten Brille an die trägerspezifischen Daten eines Brillenträgers mittels einer Schablone, die aus einem linealartigen Element (1) besteht, das mittig mit einer auf einen Zentrierpunkt (2) zulaufenden, keilförmigen, randskalierten Nasenausnehmung (3) versehen ist, wobei beidseitig des Zentrierpunktes (2) Mess-Skalen (4) zur Bestimmung des Pupillenabstandes angeordnet sind. Die Schablone zeichnet sich im Interesse einfacher Ausschneidbarkeit zur Durchführung des Verfahrens dadurch aus, daß beidendig am Element (1) dieses verlängernde, sich vom Element (1) aus gerade weiter erstreckende und in einem skalierten Abknickbereich (5) abknickbare Ohrauflagelaschen (6) angeordnet und diese an ihren Endbereichen (7) ebenfalls mit Meßskalen (8) versehen sind. Zur indirekten Anpassung einer Brille wird so vorgegangen, daß die abrufbar beim Optiker gespeicherte, Meßskalen enthaltende Brillenschablone abgerufen und die abgerufene, ein oder mehrteilige Schablonenkonfiguration auf einem Druckbogen ausgedruckt und der Schablonenausdruck ausgeschnitten wird. Danach werden mit der ausgeschnittenen Schablonenkonfiguration die trägerspezifischen Daten genommen und diese Daten an den Optiker zurückübertragen, der danach anhand dieser zurückübertragenen Daten die Anpassung der Brille vornimmt.

## Beschreibung

Die Erfindung betrifft ein Verfahren zur indirekten Anpassung einer ausgewählten Brille an die trägerspezifischen Daten eines Brillenträgers mittels einer Schablone und bezieht sich außerdem auf die Schablone selbst.

Derartige Schablonen sind als sogenannte Optikerlineale hinlänglich bekannt und in Benutzung. Bspw. sei hierzu verwiesen auf DE-A-32 47 509 C1, DE-A-35 18 215 C2, US-A-2 326 030 und WO-A-98/15222. Solche in der Regel aus Kunststoff gegf. aber auch aus Kartonage bestehenden Lineale bzw. Optikermeßgeräte haben eine Länge von ca. 15 bis 20 cm und sind bis auf den mittigen Anordnungsbereich der Nasenausnehmung ca. 2 cm breit. Um neben der groben Einschätzung bzw. Messung der Schädelbreite in Augenhöhe, insbesondere aber den weitestgehend genauen Pupillenabstand beider Augen zueinander messen zu können, werden diese Lineale, vom Optiker gehalten, mit der keilförmigen, randskalierten Nasenausnehmung auf den Nasenwurzelbereich aufgesetzt, um die Nasenwurzelbreite in etwa festhalten zu können. Ein Bezug zu den Ohren ist bei den Gegenständen der DE-A-32 47 509 und US-A-2 2 326 030 nicht herstellbar, wohl aber bei den Geräten nach der DE-A-35 18 215 und der WO-A-98/15222, bei denen es sich zum Einen um ein regelrechtes, in sich verstellbares Brillengestell handelt und zum Anderen um eine Schieberkonstruktion bspw. aus Kartonage, die insgesamt sechs verstellbare Schieber aufweist. Sofern Auswahl und Anpassung eines geeigneten Brillengestelles unmittelbar beim Optikger erfolgen, sind derartige Hilfsgeräte durchaus geeignet, um derartige Messungen und Anpassungen durchführen zu können. Anders liegen die Verhältnisse jedoch, wenn die Anschaffung einer neuen Brille nicht mehr auf direktem Wege beim Optiker erfolgt bzw. erfolgen kann, was bspw. dann der Fall sein würde, wenn sich die betreffende Person, aus welchen Gründen auch immer, nicht selbst zum Optiker begeben kann oder möchte. Jedem möglichen Brillenträger, der eine neue Brille benötigt, ein solches Hilfsgerät zur Eigenermittlung der notwendigen Daten gewissermaßen auf Verdacht zur Verfügung zu stellen, könnte zwar theoretisch in Betracht gezogen werden, wie dies bspw. mit Fußgrößenschablonen von Schuhversandhäusern praktiziert wird, damit vom Kunden selbst die erforderliche Schuhgröße auf schriftlichem Wege bestellt werden kann, ist aber im hier interessierenden Optikerbereich aus Kostengründen praktisch nicht möglich, zumal für eine fachgerechte Brillenanpassung die Mitteilung nur eines Meßwertes an den Optiker nicht ausreicht. Bei Schuhversandhäusern ist der Kostenaufwand für die Bereitstellung von Fußschablonen insofern vertretbar, als jedem Kunden nur bei einer Erstbestellung eine solche Schablone zur Verfügung gestellt werden muß, die dann immer benutzbar bleibt, ganz abgesehen davon, daß Schuhbestellungen in der Regel weitaus häufiger erfolgen als Brillenneuanschaffungen.

Der Erfindung liegt die Aufgabe zugrunde, ein Verfahren zur indirekten Anpassung einer ausgewählten Brille an die trägerspezifischen Daten eines Brillenträgers mittels einer Schablone zu schaffen und eine dafür geeignete, für den betreffenden Optiker praktisch keine kostenverursachende Schablone, gemäß dem eine zufriedenstellende Brillenanpassung ohne Gegenwart des Brillenträgers gewährleistet ist.

Bezüglich des Verfahrens ist diese Aufgabe durch die im Kennzeichen des unabhängigen Patentanspruches angeführten Merkmale gelöst. Bezgl. des Verfahrens vorteilhafte Weiterbildungen ergeben sich nach den davon abhängigen Unteransprüchen.

Eine Schablone gemäß der vorerwähnten Schablone nach der WO 98/15222 wäre dafür nicht geeignet, da aufgrund der insgesamt erforderlichen sechs Schieber viel zu kompliziert und für den potentiellen Brillenbesteller selbst nicht zumutbar und akzeptabel.
Die Lösung bzgl. der Schablone zur Durchführung des Verfahrens besteht, und zwar nach wie vor von einer Schablone ausgehend, die aus einem linealartigen Element gebildet ist, das mittig mit einer auf einen Zentrierpunkt zulaufenden, keilförmigen, randskalierten Nasenausnehmung versehen ist, wobei beidseitig des Zentrierpunktes Mess-Skalen zur Bestimmung des Pupillenabstandes angeordnet sind, erfindungsgemäß darin, daß beidendig am Element dieses verlängernde, sich vom Element aus gerade weiter erstreckende und in einem skalierten Abknickbereich abknickbare Ohrauflagelaschen angeordnet und diese an ihren Endbereichen ebenfalls mit Meßskalen versehen sind. Bzgl. vorteilhafter Weiterbildungen der Schablone selbst wird auf die darauf bezogenen abhängigen Ansprüche verwiesen, wobei jedoch vorab insbesondere darauf hingewiesen sei, daß die Schablone, da sie für eine Selbstausmessung durch den Brillenträger bestimmt ist, mit numerische Zahlenangaben zu den Skalen spiegelverkehrt versehen ist, d.h., vor einem Spiegel stehend, werden Skalenwerte an der Schablone für den Benutzer problemlos direkt ablesbar. Schließlich besteht für den vorliegenden Zweck eine vorteilhafte Ausführungsform darin, daß die Schablone mit ihren Teilen und Skalierungen als integrale Teile eines Ausschnittbogens ausgebildet ist.

Das erfindungsgemäße Verfahren und die Schablone einschließlich deren vorteilhafter Weiterbildungen werden nachfolgend anhand der zeichnerischen Darstellung von Ausführungsbeispielen näher erläutert.

Es zeigt
- Fig.1: perspektivisch die Schablone im aufsetzbaren Zustand;
- Fig.2: in gegenüber Normalgröße etwas verkleinerter Ansicht die neuartige Schablone;
- Fig.3: in Ansicht eine besondere Ausführungsform der Schablone;
- Fig.4: in Draufsicht eine besondere Einzelheit der Schablone;
- Fig.5: in Vorderansicht eine weitere besondere Ausführungsform
- Fig.6: in Ansicht eine weitere besondere Ausführungsform der Schablone und
- Fig.7: zum Vergleich das Beispiel eines bekannten und einfachen Optikerlineals.

Die zunächst zu beschreibende Schablone besteht nach wie vor und unter Verweis auf die Vergleichsfig.7 aus einem linealartigen Element 1, das mittig mit einer auf einen Zentrierpunkt 2 zulaufenden, keilförmigen, randskalierten Nasenausnehmung 3 versehen ist, wobei beidseitig des Zentrierpunktes 2 Mess-Skalen 4 zur Bestimmung des Pupillenabstandes angeordnet sind.

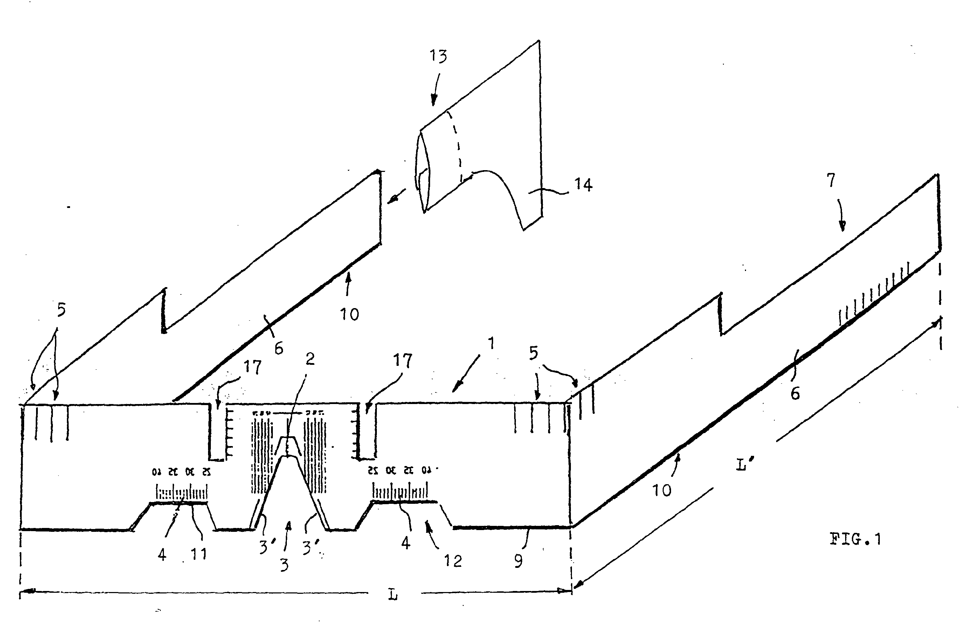

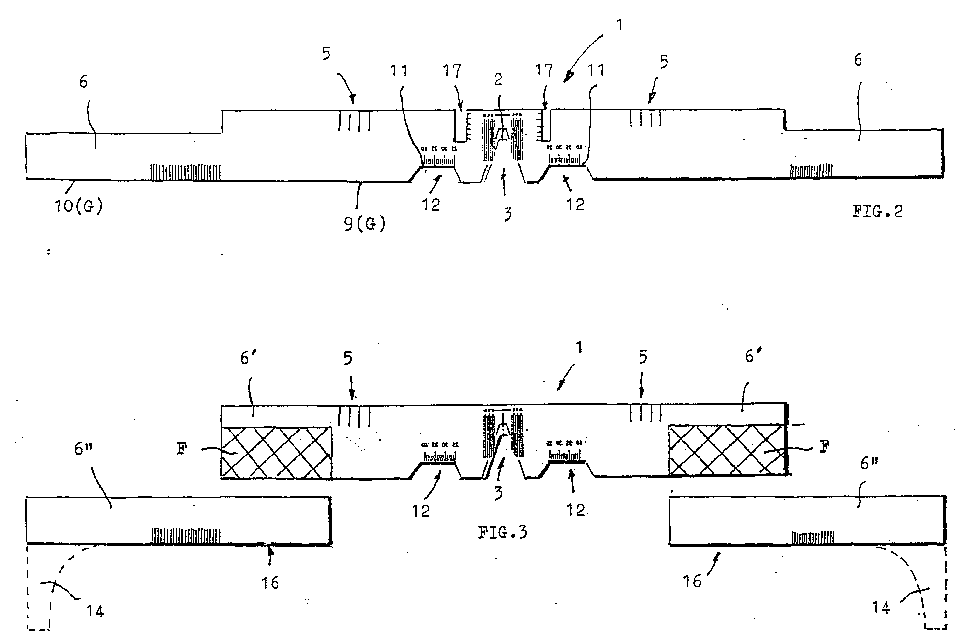

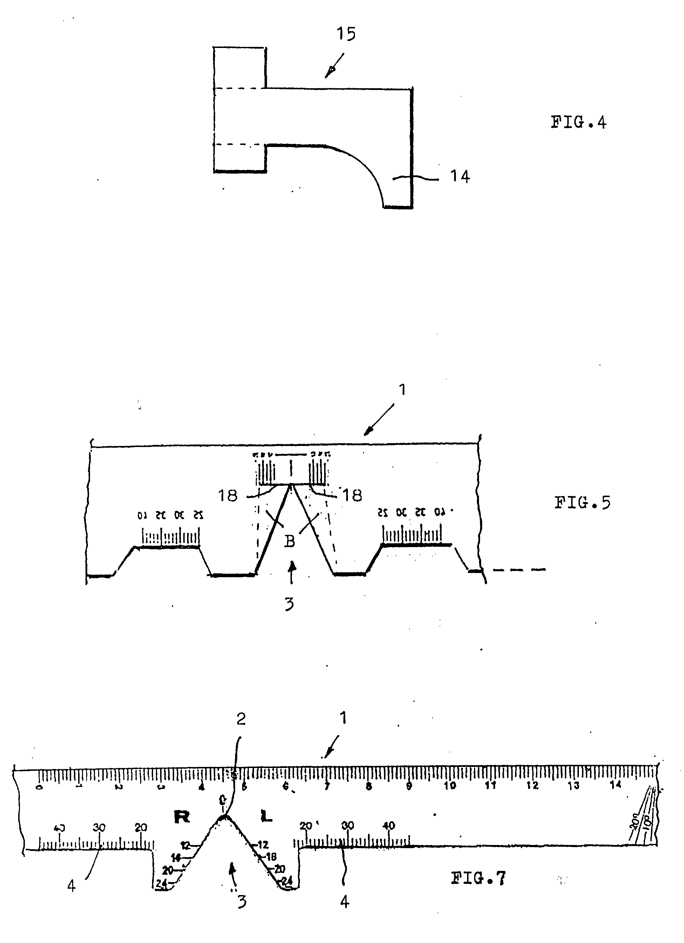

Für die erfindungsgemäße Schablone ist nun unter Bezug auf Fig.2 wesentlich, daß beidendig am Element 1 dieses verlängernde, sich vom Element 1 aus gerade weiter erstreckende und in einem skalierten Abknickbereich 5 abknickbare Ohrauflagelaschen 6 angeordnet und diese an ihren Endbereichen 7 ebenfalls mit Maßskalen 8 versehen sind. Verwiesen wird hierzu auch auf Fig.1, die die Schablone mit in den skalierten Abknickbereichen 5 abgeknickten Ohrauflagelaschen 6, d.h. im aufsetzbaren Zustand, darstellt.
Das linealartige Element 1 mit seinen Ohrauflagelaschen 6 ist dabei aus ausreichend steifen Papier oder auch aus einer dünnen bieg- bzw. knickbaren Kartonage gebildet, und zwar insbesondere auch mit Rücksicht darauf, daß die Schablone, entsprechend vorgedruckt aus einem Papier- oder Kartonagenzuschnitt in geeigneter Weise ausgeschnitten werden kann.
Das Element 1 und die Ohrauflagelaschen 6 sind mit ihren Unterkanten 9,10 sich auf einer Geraden G erstreckend angeordnet. Dadurch können die Auflagelaschen 6 problemlos und, wie einleitend vorerwähnt, korrekt rechtwinklig zum Element 1 abgeknickt werden, wie dies in Fig.1 dargestellt ist.
Die beiden Ohrauflagelaschen 6 sind mit einer Länge L' bemessen, die jeweils im wesentlichen der Länge L des Elementes 1 entspricht. Damit ist die Länge L' auf das notwendige Maß beschränkt und garantiert eine Auflage auf dem oberen Ohransatz am Kopf, da der Ohrenabstand zur Gesichtsfläche in Augenhöhe normalerweise kleiner ist als die Kopfbreite in dieser Höhe.
Aus den einleitend genannten Gründen sind die Pupillenabstandsskalierungen 4 längs sich zur vorerwähnten Geraden G parallel erstreckender Begrenzungsränder 11 von zur Geraden G hin offenen Sichtausnehmungen 12 angeordnet, wie dies aus den Fig.1 bis 3 ersichtlich ist.
Bezüglich der Ohrauflagelaschen 6 kann man vorteilhaft noch einen Schritt weitergehen und an diesen Schiebehülsen 13 anordnen bzw. aufschieben, die mit ohrbügelartigen Laschen 14 versehen sind. Eine dieser Schiebehülsen 13 ist perspektivisch in Fig.1 mit dargestellt. Unter Verweis auf Fig.4 sind diese Schiebehülsen 13 aus einem entsprechend gefalteten Papier- oder Kartonagenzuschnitt 15 gebildet, welche Zuschnitte 15 auch die ohrbügelartigen Laschen 14 mit enthalten.
Unter Verweis auf Fig.3 sind die Ohrauflagelaschen 6 jeweils aus zwei Teilen 6',6" gebildet, von denen die ersten Teile 6' die beidendigen Verlängerungen des Elementes 1 bilden und die zweiten Teile 6" als separate Papier- oder Kartonagenzuschnitte 16 am jeweils ersten Teil 6' befestigt sind. Diese Befestigung erfolgt bspw. mittels eines Schnellklebers an den hier kreuzschraffiert dargestellten, mit aufgedruckten Feldern F der ersten Teile 6'. Diese Ausbildung bzw. Ausführungsform hat ihren Grund insbesondere darin, daß ein Schablonenzuschnitt gemäß Fig.2 eine Gesamtlänge und orientiert an normalen Schädelabmessungen von ca. 45 cm haben muß. Teilt man dagegen die Schablone im Sinne der Fig.3 auf, reduziert sich der Mittelteil bzw. das Element 1 mit den Teilen 6' auf eine Länge, die noch auf einem gängigen DIN A4 Format unterbringbar ist. Dies wiederum ist insofern von Interesse, als damit eine solche Schablone bspw. aus dem Internet heruntergeladen und in Normalgröße auf gängigem DIN-A4 Format ausgedruckt werden kann und damit die Schablone mit ihren Teilen und Skalierungen als integrale Teile eines Ausschnittbogens zur Verfügung stehen und ausgeschnitten werden können. Ein solcher Ausschnittbogen würde also bspw. die in den Fig.3,4 enthaltenen Teile umfassen, d.h., den Zuschnitt 15 gemäß Fig.4 natürlich zweifach.
Im übrigen können die separaten Teile 6", wie gestrichelt in Fig.3 mit angedeutet, ebenfalls mit ohrbügelartigen Laschen 14 versehen sein. Schiebehülsen 13 wären in diesem Falle entbehrlich.
Besonderheiten sind insofern noch an der erfindungsgemäßen Schablone vorgesehen, als zum einen numerische Zahlenangaben zu den Skalen spiegelverkehrt an der Schablone angeordnet sind, wie insbesonder in Fig.1 an den Skalierungen für die Pupillenabstände ersichtlich, und zum anderen, wie ebenfalls in Fig.1,2 mit dargestellt, können im Element 1 beidseitig benachbart zu zur Nasenausnehmung 3 ebenfalls skalierte Schlitze 17 vorgesehen sein, mit denen Höhe und vertikale Breite der Augenbrauen ermittelbar sind.

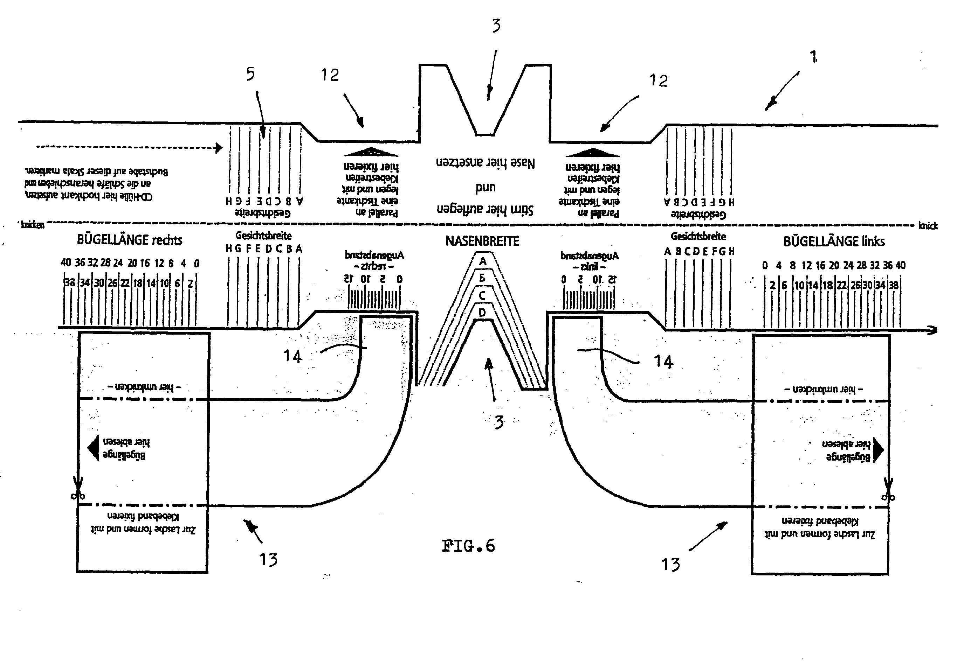

Zu der skalierten Nasenausnehmung 3 sei noch bemerkt, daß dafür zu den Ausnehmungsrändern 3' parallele Linien 3" mit aufgedruckt sind (siehe Fig.1), längs der in Anpassung an die tatsächliche Nasenwurzelbreite dank des Schablonenmaterials Ein- und Abschnitte vorgenommen werden können, um auf diese Weise einen zumindest angenähert genauen Paßsitz auf der Nasenwurzel zu erreichen, insbesondere aber die skalierten Begrenzungsränder 11 der Sichtausnehmungen 12 möglichst genau auf den sogenannten Pupillendurchblickpunkt auszurichten.
Bezgl. der Anpaßbarkeit an die Nasenwurzelbreite besteht unter Verweis auf Fig.5 eine weitere Ausführungsform darin, daß ausgehend vom Zentrierpunkt 2 und parallel zur Geraden G Einschnitte 18 im Element 1 angeordnet sind. Entsprechende Biegsamkeit des Schablonenmaterials vorausgesetzt, können sich dadurch die Bereiche B mehr oder weniger stark nach vorn, d.h. zum Nasenrücken hin, aufbiegen und sich damit an die tatsächliche Nasenwurzelbreite anpassen, für welche Ausbiegung dann die Nasenwurzelskalierung maßgebend ist.
Eine besonders vorteilhafte Ausführungsform der Schablone ist in Fig.6 dargestellt, wonach die Schablonenkonfiguration zu einer sich über die Länge der Schablone erstreckenden Faltlinie spiegelbildlich ausgebildet ist und in dieser Form abrufbar bereitgehalten wird. Die entsprechend abgerufene, auf den Druckbogen übernommene Schablonenkonfiguration wird dabei nach ihrem Ausschnitt längs der Faltlinie gefaltet und zusammengeklebt wird. Zusammen mit dem Klebstoff erhält dabei die Schablone schon eine ausreichende Steifigkeit und Stabilität, d.h., ein Aufkleben des Druckbogens auf steiferes Material als Papier und dessen anschließendes Ausschneiden sind entehrlich.

Wie aus den Fig.2,3 und 6 ersichtlich, ist die erfindungsgemäße Schablone auf eine sehr einfache, promblemos ausschneidbare Konfiguration reduziert, was schon einmal wesentliche Voraussetzung dafür ist, den Benutzer verfahrensgemäß in den Besitz einer solchen Schablone zu bringen, die Daten zu ermitteln und diese Daten in geeigneter Weise wiederum dem Optiker zur Verfügung zu stellen.

Bei den erfindungsgemäßen Verfahrensmaßgaben, nämlich eine abrufbar beim Optiker gespeicherte, Meßskalen enthaltende Brillenschablone abzurufen und die abgerufene, ein oder mehrteilige Schablonenkonfiguration, wie vorbeschrieben, auf einem Druckbogen auszudrucken und den Schablonenausdruck auszuschneiden, danach mit der ausgeschnittenen Schablonenkonfiguration die trägerspezifischen Daten zu ermitteln und diese Daten an den Optiker zurückzuübertragen und danach anhand dieser zurückübertragenen Daten die Anpassung der Brille vorzunehmen, handelt es sich um rein technische Schritte, die keiner besonderen Erläuterung bedürfen, da sich diese, soweit es sich um das Abrufen und das Ausdrucken handelt, mit den heute bei praktisch jederman verfügbaren Gerätschaften durchführen lassen.

## Patentansprüche

1. Verfahren zur indirekten Anpassung einer ausgewählten Brille an die trägerspezifischen Daten eines Brillenträgers mittels einer Schablone,
**dadurch gekennzeichnet,**
daß eine abrufbar beim Optiker gespeicherte, Meßskalen enthaltende Brillenschablone abgerufen und die abgerufene, ein oder mehrteilige Schablonenkonfiguration auf einem Druckbogen ausgedruckt und der Schablonenausdruck ausgeschnitten wird,
daß danach mit der ausgeschnittenen Schablonenkonfiguration die trägerspezifischen Daten genommen und diese Daten an den Optiker zurückübertragen und danach anhand dieser zurückübertragenen Daten die Anpassung der Brille vorgenommen wird.

2. Verfahren nach Anspruch 1,
**dadurch gekennzeichnet,**
**daß** der Schablonenausdruck auf ein geeignetes, ausschneidbares, ausreichend biegesteifes Material übertragen und aus diesem die Schablonenkonfiguration ausgeschnitten wird.

3. Verfahren nach Anspruch 2,
**dadurch gekennzeichnet,**
**daß** zur Übertragung der ausgedruckten Schablonenkonfiguration der diese enthaltende Druckbogen auf das ausschneidbare Material aufgeklebt wird.

4. Verfahren nach Anspruch 1,
**dadurch gekennzeichnet,**
**daß** die Schablonenkonfiguration zu einer sich über die Länge der Schablone erstreckenden Faltlinie spiegelbildlich abrufbar bereitgehalten und die entsprechend abgerufene, auf den Druckbogen übernommene Schablonenkonfiguration nach ihrem Ausschnitt längs der Faltlinie gefaltet und zusammengeklebt wird.

5. Schablonenkonfiguration zur Durchführung des Verfahrens nach einem der Ansprüche 1 bis 4 und zur Bestimmung individueller Gesichtsmaße als Maßvorgaben für die Auswahl und Anpassung individueller Brillengestelle, bestehend aus einem linealartigen Element (1), das mittig mit einer auf einen Zentrierpunkt (2) zulaufenden, keilförmigen, randskalierten Nasenausnehmung (3) versehen ist, wobei beidseitig des Zentrierpunktes (2) Mess-Skalen (4) zur Bestimmung des Pupillenabstandes angeordnet sind,
**dadurch gekennzeichnet,**
**daß** beidendig am Element (1) dieses verlängernde, sich vom Element (1) aus gerade weiter erstreckende und in einem skalierten Abknickbereich (5) abknickbare Ohrauflagelaschen (6) angeordnet und diese an ihren Endbereichen (7) ebenfalls mit Meßskalen (8) versehen sind.

6. Schablone nach Anspruch 5,
**dadurch gekennzeichnet,**
**daß** das Element (1) und die Ohrauflagelaschen (6) mit ihren Unterkanten (9,10) sich auf einer Geraden (G) erstreckend angeordnet sind.

7. Schablone nach Anspruch 5 oder 6,
**dadurch gekennzeichnet,**
**daß** die beiden Ohrauflagelaschen (6) mit einer Länge (L') bemessen sind, die jeweils im wesentlichen der Länge (L) des Elementes (1) entspricht.

8. Schablone nach einem der Ansprüche 5 bis 7,
**dadurch gekennzeichnet, daß** Pupillenabstandsskalierungen (4) längs sich zur Geraden (G) parallel erstreckender Begrenzungsränder (11) von zur Geraden (G) hin offenen Sichtausnehmungen (12) angeordnet sind.

9. Schablone nach einem der Ansprüche 5 bis 8,
**dadurch gekennzeichnet,**
**daß** an den Ohrauflagelaschen (6) Schiebehülsen (13) angeordnet und diese mit ohrbügelartigen Laschen (14) versehen sind.

10. Schablone nach Anspruch 9,
**dadurch gekennzeichnet,**
**daß** die Schiebehülsen (13) aus einem entsprechend gefalteten Papier- oder Kartonagenzuschnitt (15) gebildet sind, welche Zuschnitte (15) auch die ohrbügelartigen Laschen (14) mit enthalten.

11. Schablone nach einem der Ansprüche 5 bis 8,
**dadurch gekennzeichnet,**
**daß** die Ohrauflagelaschen (6) jeweils aus zwei Teilen (6',6") gebildet sind, von denen der eine erste Teil (6') die Verlängerung des Elementes (1) bildet und der zweite Teil (6") als separater Papier- oder Kartonagenzuschnitt (16) am ersten Teil (6') befestigt ist.

12. Schablone nach einem der Ansprüche 5 bis 11,
**dadurch gekennzeichnet,**
**daß** numerische Zahlenangaben zu den Skalen spiegelverkehrt an der Schablone angeordnet sind.

13. Schablone nach einem der Ansprüche 5 bis 12,
**dadurch gekennzeichnet,**
**daß** die Schablone mit ihren Teilen und Skalierungen als integrale Teile eines Ausschnittbogens ausgebildet sind.
